Europäisches Patentamt

European Patent Office

Office européen des brevets

(19)

(11) Numéro de publication : **0 319 369 B1**

(12) **FASCICULE DE BREVET EUROPEEN**

(45) Date de publication du fascicule du brevet :
25.03.92 Bulletin 92/13

(51) Int. Cl.$^5$ : **C07C 2/78,** C07C 4/04,
B01J 19/24

(21) Numéro de dépôt : **88402913.3**

(22) Date de dépôt : **21.11.88**

(54) **Procédé et appareil pour la conversion des hydrocarbures.**

Le dossier contient des informations
techniques présentées postérieurement au
dépôt de la demande et ne figurant pas dans le
présent fascicule.

(30) Priorité : **03.12.87 FR 8716804**

(43) Date de publication de la demande :
**07.06.89 Bulletin 89/23**

(45) Mention de la délivrance du brevet :
**25.03.92 Bulletin 92/13**

(84) Etats contractants désignés :
**AT BE CH DE ES GB GR IT LI LU NL SE**

(56) Documents cités :
**US-A- 4 672 144**

(73) Titulaire : **GAZ DE FRANCE (SERVICE
NATIONAL)
23 rue Philibert-Delorme
F-75017 Paris (FR)**

(72) Inventeur : **Come, Guy-Marie
9, rue Winston Churchill
F-54000 Nancy (FR)**

(74) Mandataire : **Lerner, François et al
LERNER & BRULLE S.C.P. 5, rue Jules
Lefèbvre
F-75009 Paris (FR)**

EP 0 319 369 B1

Jouve, 18, rue Saint-Denis, 75001 PARIS

## Description

La présente invention se rapporte à un procédé et à un appareil pour la conversion des hydrocarbures, tels que du méthane, destinés à la fabrication de produits de transformation, tels que l'acétylène.

L'objet visé est, en fait, de convertir des matières premières hydrocarbonées en produits de base destinés en particulier à l'industrie chimique.

Parmi les matières premières ou charges hydrocarbonées pouvant être retenues dans le cadre de l'invention, on notera, outre le méthane, les gaz naturels, les gaz de pétrole liquéfiés (GPL), les essences légères et naphtées, les fractions lourdes du pétrole ou issues de son raffinage, le pétrole brut et, plus généralement, les hydrocarbures gazeux et liquides obtenus soit à partir de matières premières fossiles, schistes et sables bitumineux, charbon, soit à partir de la biomasse, de matières d'origine animale ou de différents déchets (ordures ménagères, ...).

Quant aux produits de base qui sont obtenus par conversion de ces matières premières et qui sont actuellement utilisés largement dans l'industrie chimique, il s'agit soit d'hydrocarbures insaturés de faible poids moléculaire, tels que l'acétylène, les oléfines, di-oléfines, soit des hydrocarbures saturés, également de poids moléculaire relativement faible, soit encore éventuellement de l'oxyde de carbone ou de l'hydrogène.

Il existe actuellement différents types de procédés et d'appareils de conversion des hydrocarbures.

On peut tout d'abord citer les procédés dits "thermiques". Ces procédés peuvent être classés en deux grandes catégories.

La première est celle où l'énergie nécessaire à la réaction de conversion est apportée par une source électrique haute puissance (arc électrique ou plasma).

Dans la seconde catégorie, l'énergie est d'origine chimique et est obtenue par la combustion d'une partie de la charge hydrocarbonée elle-même (procédés connus sous l'appellation "en un stade") ou d'un autre combustible, les produits de combustion obtenus pouvant être ensuite soit mélangés à l'hydrocarbure (procédés dits "en deux stades"), soit utilisés pour chauffer des matériaux réfractaires équipant une paroi de la chambre de réaction, de façon à assurer la conversion de ce même hydrocarbure (procédé dit "cyclique").

Les deux caractéristiques essentielles des procédés connus en un stade consistent, d'une part, dans le préchauffage des produits que l'on va faire réagir (à savoir au moins de l'oxygène, et un type d'hydrocarbure), à l'aide d'une réaction de combustion auxiliaire et, d'autre part, dans le prémélange de ces mêmes produits avant la réaction exothermique d'oxydation qui va conduire à la conversion de l'hydrocarbure de départ.

Dans ces procédés en un stade, le prémélange des produits, dans une chambre prévue à cet effet, est réputé indispensable, la vitesse de diffusion des gaz étant considérée comme incompatible avec la durée de contact des produits entre-eux nécessairement limitée pour assurer la réaction de conversion dans des conditions convenables.

Si l'on s'intéresse de plus près à l'évolution des réactions dans ce type de procédé, on note que l'hydrocarbure subit pour partie une conversion ( en acétylène s'il s'agit du méthane) et, pour partie, une transformation en oxyde de carbone, hydrogène, gaz carbonique et eau. La formation de suies est pratiquement inévitable et constitue un inconvénient difficile à surmonter. La trempe ultérieure nécessaire du mélange réactionnel résultant permet, lorsqu'elle est effectuée à l'huile, de récupérer une partie non négligeable de la chaleur sensible des gaz après réaction tout en stabilisant les produits de conversion obtenus. Par contre, la sélectivité est médiocre, c'est-à-dire qu'un taux relativement important d'oxyde de carbone est produit.

Dans les procédés en deux stades, le premier stade consiste à produire un gaz à très haute température par une réaction de combustion du type hydrogène-oxygène. Au niveau du second stade, le gaz chaud obtenu est mélangé à l'hydrocarbure que l'on veut convertir et qui subit alors une réaction de pyrolyse, conduisant à des produits de pyrolyse (tels que l'acétylène et l'éthylène). Ensuite les produits sont trempés, souvent par une trempe (directe) à l'huile.

En pratique, ce type de procédé est apparu comme présentant certains inconvénients, tels en particulier celui de la réalisation en matériaux hautement résistants en température (matériaux réfractaires) des parois de l'appareil dans lequel se déroule la réaction, compte tenu du caractère particulièrement exothermique et violent de la réaction.

L'invention a justement pour objet de résoudre les difficultés et inconvénients des différents procédés de conversion actuellement connus. Ce but est atteint dans l'invention qui se caractérise tout d'abord en ce que :

– on utilise en tant que substances de départ au moins un type d'hydrocarbure et un premier gaz contenant au moins environ 20 % d'oxygène en volume,

– on fait circuler ces substances indépendamment l'une de l'autre et suivant des flux à co-courants, sans mélange, en adoptant une répartition spatiale telle que ledit flux d'hydrocarbure soit situé autour du flux dudit premier gaz,

– on laisse ces mêmes substances se mélanger à un premier niveau donné de circulation et on enflamme le mélange d'oxygène et d'hydrocarbure de manière à induire la réaction de conversion,

– on conduit une trempe du mélange résultant converti, à partir d'un second niveau de circulation situé en aval du premier,

– et on récupère les produits de conversion résultants trempés.

Dans certains cas et notamment de façon à améliorer la sélectivité des réactions, il sera néanmoins avantageux de prévoir, en outre, comme corps de départ, un second gaz contenant au moins 30 % d'hydrogène en volume. On fera alors circuler, tout d'abord sans qu'il y ait mélange, ce second gaz entre les flux d'hydrocarbure et dudit premier gaz à teneur en oxygène, et, au niveau de circulation précité où l'on vient enflammer l'hydrocarbure et l'oxygène, et tout en laissant les corps se mélanger, on enflammera également l'hydrogène.

Dans ce cas, le corps obtenu par le mélange et la réaction de combustion du premier gaz "riche" en oxygène et du second gaz "riche" en hydrogène sera mélangé, en quelque sorte "en l'état", à l'hydrocarbure qui entoure ce second gaz, et ce sans que de la vapeur d'eau ait été ajoutée préalablement, avant inflammation des corps.

En outre, dans ce cas la réaction du premier gaz s'effectuera largement sans contact avec l'hydrocarbure, par suite de l'interposition du second gaz entre les deux. On notera également que le mélange des deux gaz et de l'hydrocarbure s'effectuera largement par diffusion, caractéristique considérée jusqu'à présent (et comme on l'a vu) comme rédhibitoire.

Il est à noter que dans l'invention, le premier gaz riche en oxygène pourra contenir une certaine quantité d'hydrogène.

Selon une autre caractéristique de l'invention, pour tremper ledit mélange converti résultant à partir dudit second niveau de circulation, on conduira avantageusement un échange thermique indirect, sans qu'il y ait mélange, entre ce mélange résultant converti et une partie au moins desdites substances de départ que l'on fera circuler, à contre-courant, autour dudit mélange résultant converti, de manière, pour assurer ladite trempe, à préchauffer lesdites substances avant qu'elles ne circulent en flux à co-courants suivant l'étape b) de la revendication 1. Pour compléter cette "première" trempe par échange thermique indirect, on pourra injecter également un fluide de trempe dans le mélange résultant converti.

Comme on l'a déjà noté l'invention se rapporte également à un appareil pour la mise en oeuvre du procédé que l'on vient de présenter.

Cet appareil qui a donc pour but de conduire une réaction de conversion exothermique entre au moins un premier et un second réactifs (tels qu'un hydrocarbure et un premier gaz contenant de l'oxygène) se caractérise en particulier en ce qu'il comprend :

– une chambre de mélange et de réaction pour y mélanger et y enflammer au moins lesdits premier et second réactifs de manière à conduire la réaction exothermique de conversion, ladite chambre étant limitée extérieurement par une paroi réalisée au moins partiellement en matériau thermiquement conducteur, pour transférer la chaleur de la réaction de conversion à travers cette paroi,

– des moyens d'allumage pour induire la réaction de conversion au moins entre lesdits premier et second réactifs,

– un premier conduit d'alimentation communiquant avec la chambre de mélange et de réaction pour y introduire, à une première extrémité, ledit premier réactif,

– un second conduit d'alimentation communiquant avec ladite chambre de mélange et de réaction pour introduire le second réactif à cette même première extrémité, ce second conduit d'alimentation s'étendant extérieurement le long dudit premier conduit, sensiblement parallèlement à ce dernier, au moins à proximité de ladite chambre de mélange et de réaction,

– des conduits de récupération communiquant avec la chambre de mélange et de réaction pour récupérer les produits de réaction résultants,

– des chambres de préchauffage communiquant avec l'un au moins desdits premier et second conduits d'alimentation et étant alimentées avec l'un au moins desdits réactifs, ces chambres de préchauffage s'étendant extérieurement au contact de ladite paroi extérieure thermiquement conductrice de la chambre de mélange et de réaction pour préchauffer le, ou les réactif(s) circulant dans ces chambres et pour fournir à la chambre de mélange et de réaction une première zone de trempe interne, par transfert de chaleur de la réaction de conversion vers le ou les, réactif(s) circulant dans lesdites chambres de préchauffage, avant que lesdits réactifs soient admis dans les conduits d'alimentation.

Grâce à la répartition spatiale particulière des différents corps (laquelle assure une diminution des contraintes thermiques dans l'appareil), vers l'entrée de la chambre de mélange et de réaction, les parois de la chambre de réaction-mélange pourront être métalliques. Et le caractère thermiquement conducteur de ces parois pourra être mis à profit pour assurer concomitamment le préchauffage des réactifs avant leur introduction dans la chambre de réaction et la trempe initiale des produits en réaction dans cette chambre, bien entendu par transfert de chaleur à travers lesdites parois.

Selon une autre caractéristique importante de cet appareil, le rapport de la surface mouillée sur le volume de la chambre de mélange et de réaction sera de préférence compris entre 40 et 250 $m^{-1}$, le mélange s'effectuant alors largement par diffusion.

Les objets, caractéristiques et avantages de l'invention apparaîtront encore plus clairement de la description qui va suivre faite en référence aux dessins d'accompagnement dans lesquels :

– la figure 1 est une vue partielle du réacteur conforme à l'invention, selon une coupe transversale médiane le long de la ligne I-I de la figure 5,

– la figure 2 est une vue en coupe des conduits d'alimentation du réacteur selon la ligne II-II de la figure 1,

– la figure 3 est une vue en coupe du réacteur selon la ligne III-III de la figure 1,

– la figure 4 est une forme possible de réalisation de l'extrémité des différents conduits précitées, au niveau où ils débouchent sur la chambre de mélange/réaction, suivant une demi-coupe selon le plan de la figure 1,

– et la figure 5, est une vue schématique extérieure de l'appareil de l'invention.

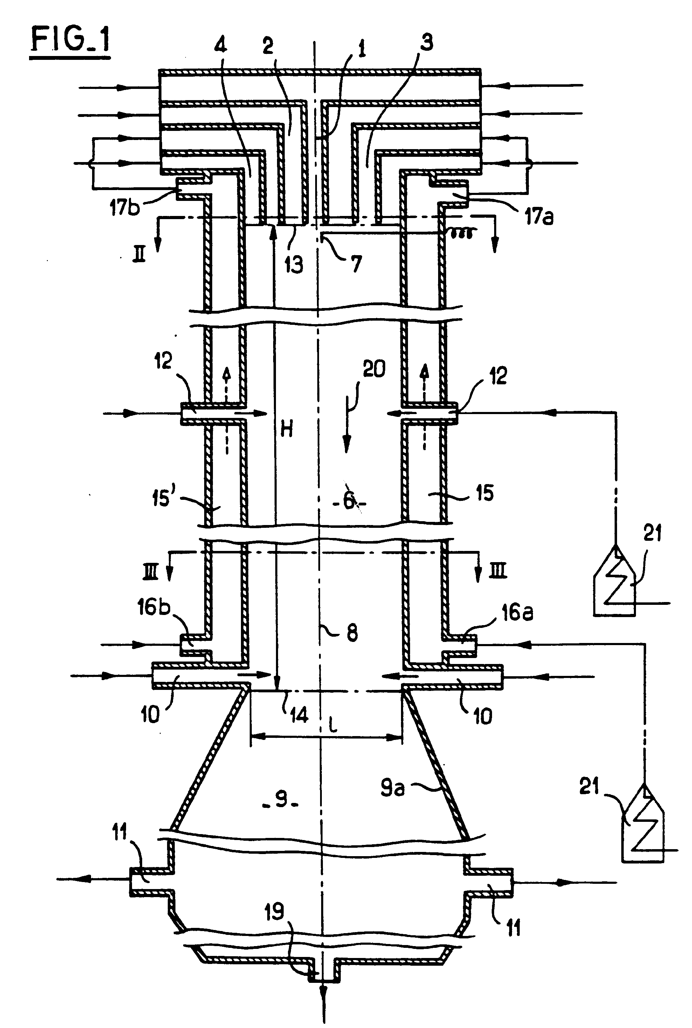

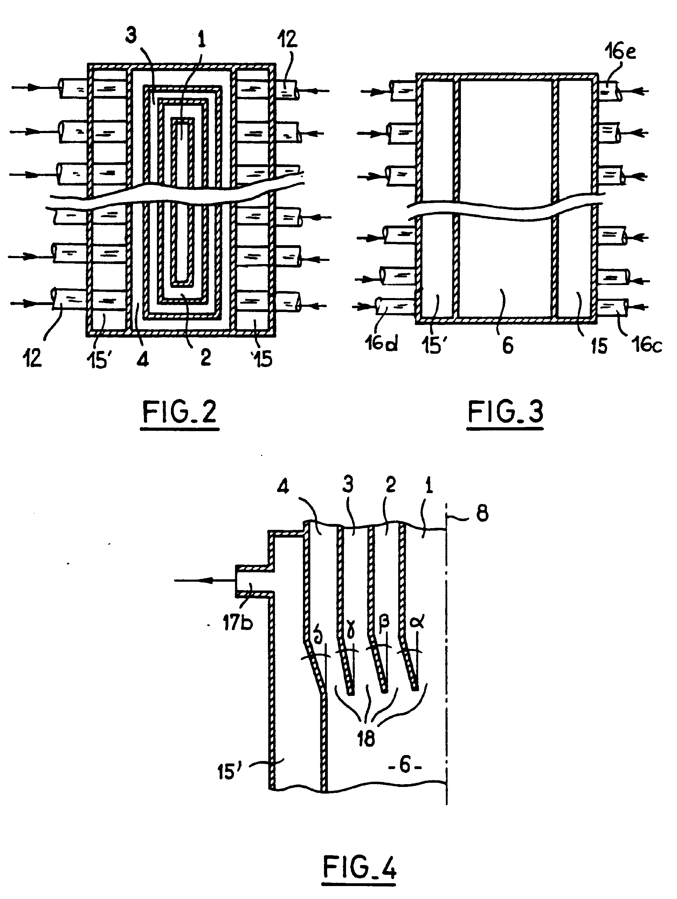

Si l'on se reporte tout d'abord aux figures 1 et 2, on voit que le réacteur de l'invention comprend essentiellement une chambre 6 de mélange/réaction d'axe général vertical 8 qui se prolonge dans son axe et à sa partie inférieure par une autre chambre 9 dite chambre de trempe, laquelle se raccorde à la chambre 6 par un divergent ou diffuseur 9a.

Conformément à l'invention, la chambre 6 de mélange/réaction est alimentée vers son sommet par au moins un premier gaz contenant au minimum 20 % d'oxygène et par une charge hydrocarbonée débouchant de veines 1, 3 d'alimentation séparées disposées sensiblement parallèlement, l'une dans l'autre, au moins à proximité de l'endroit où elles s'ouvrent sur la chambre en question.

Bien que ce mode de réalisation à deux veines soit tout à fait envisageable, il n'a pas été représenté en tant que tel. En effet, sur les différentes figures on voit qu'entre les veines 1 et 3 est interposée une veine repérée 2 destinée à alimenter la chambre 6 en un second gaz contenant au moins 30 % d'hydrogène.

On voit également qu'autour de la veine 3 d'alimentation en charge hydrocarbonée à convertir pourra être disposée une quatrième veine repérée 4. Cette dernière veine a pour objet d'alimenter le réacteur en eau, afin en particulier de favoriser le maintien à un niveau acceptable des températures de paroi de cette chambre 6.

Par souci de clarté, le reste de la description sera faite en référence à ce mode particulier de réalisation à quatre veines.

Conformément à celui-ci, la répartition spatiale des diverses alimentations du réacteur, en partie haute de la chambre 6, est donc telle que les veines s'étendent sensiblement parallèlement à l'axe 8 du réacteur, les unes dans les autres avec, de l'intérieur vers l'extérieur

– la veine 1 dans laquelle circule le premier gaz "riche" (au moins 20 %) en oxygène

– puis la veine 2 dans laquelle circule le second gaz "riche" (au moins 30 %) en hydrogène

– puis la veine 3 dans laquelle circule la charge hydrocarbonée à convertir

– puis, bien qu'elle soit facultative, la veine 4 d'alimentation en eau.

Afin d'enflammer les différents corps qui sont admis dans la chambre 6 et induire ainsi l'opération de conversion, des moyens électriques 7 d'allumage alimentés sous haute tension adaptée sont prévus vers la partie supérieure de cette chambre.

Vers la partie intermédiaire de cette même chambre, on remarquera également des canaux ou conduits 12 permettant d'introduire sensiblement perpendiculairement à l'axe 8 du réacteur une éventuelle charge hydrocarbonée complémentaire sur laquelle on reviendra ultérieurement. On notera néanmoins dès à présent que cette charge sera d'un type différent de celle qui est introduite par la veine 3.

En partie basse de la chambre 6 des buses 10 débouchant transversalement à l'axe 8, au niveau de la jonction entre la chambre et le divergent 9a, assureront en outre l'alimentation en fluide de trempe, tel par exemple que de l'huile.

Des conduits 11 permettront ensuite de récupérer et d'évacuer les produits de conversion, après qu'ils aient été trempés et donc stabilisés. Comme connu en soi, le fluide de trempe pourra, quant à lui, être évacué par le collecteur 19.

Par ailleurs, figure 4, on remarquera que les différentes veines,à proximité de l'endroit où elles débouchent sur la chambre 6,pourront présenter une partie extrême allant en se rétrécissant progressivement vers la chambre avec un angle sensiblement constant, de sorte à former un col ou goulot 18. En pratique, les angles $\alpha$, $\beta$, $\gamma$, $\delta$ de restriction des diverses veines seront adaptés aux conditions de circulation des corps entrant dans le réacteur et aux conditions de fonctionnement.

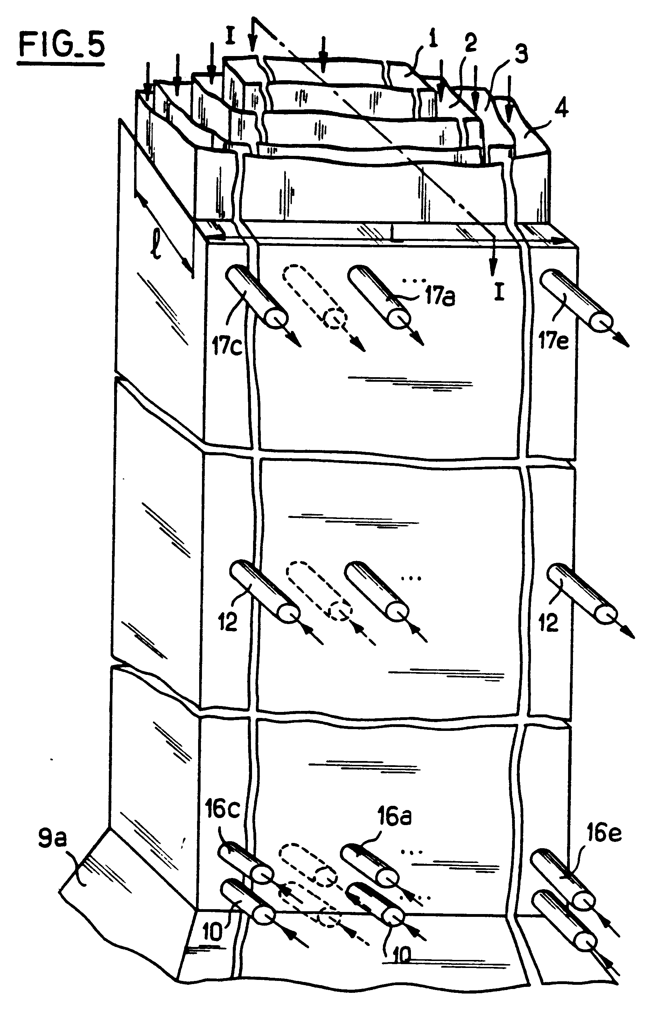

Interessons-nous maintenant plus précisément aux figures 1, 3 et 5 pour remarquer la forme et les dimensions particulières du réacteur de l'invention et plus particulièrement de la chambre 6 de mélange/réaction de forme sensiblement cylindrique que l'on a illustrée avec une section rectangulaire (figure 3). Figure 5, le réacteur n'a pas été représenté à l'échelle car selon l'invention, le rapport surface mouillée/volume de la chambre 6 sera de préférence compris entre 40 et 250m$^{-1}$.

A titre d'exemple, on pourrait envisager une telle chambre avec une largeur $\underline{l}$ de l'ordre de 2 à 8 x 10$^{-2}$ m, une longeur $\underline{L}$ de l'ordre de 8 à 10 m et une hauteur $\underline{H}$ d'environ 4 à 5 m, cette hauteur étant mesurée entre une limite supérieure 13 de la chambre située au niveau du débouché des veines d'alimentation et une limite inférieure 14, au niveau de laquelle la chambre 6 se raccorde au divergent 9a. Le rapport surface mouillée (2 x $\underline{H}$ x $\underline{L}$) / volume ($\underline{l}$ x $\underline{H}$ x $\underline{L}$) serait alors de 100 m$^{-1}$ environ.

Figure 1, on remarquera également que sur la plus grande partie de sa hauteur et au moins de part et d'autre, sur sa longueur L, la chambre 6 pourra être entourée par deux chambres de préchauffage 15, 15′ de sorte que tout ou partie des corps amenés par les veines 1-4 puissent être préchauffés par circulation à contre-courant des corps en réaction, au contact de la paroi périphérique chaude de la chambre 6 (laquelle comme on le verra pourra être métallique).

En pratique, ce sera essentiellement la charge hydrocarbonée qui sera ainsi préchauffée. Cette charge amenée par la veine 3 alors indépendante des autres veines et que l'on pourra dissocier en plusieurs canaux 16a, 16b... de façon à répartir le flux de charge sur la longueur L de la chambre, sera dans ce cas admise vers la base des chambres 15, 15′ de préchauffage, puis circulera librement sur leur hauteur, avant d'être récupérée par les canaux 17a, 17b... lesquels se réunissent ensuite pour former à nouveau une veine 3 unique.

Bien entendu, un préchauffage de ce type pour des corps autres que la charge hydrocarbonée serait réalisé selon le même principe. Il faudrait toutefois alors cloisonner les chambres 15, 15′ de préchauffage afin d'éviter tout mélange des corps avant leur introduction dans le réacteur.

Quoi qu'il en soit un tel préchauffage favorisera les conditions de fonctionnement du réacteur dont on va maintenant préciser le mode opératoire.

Comme on l'a vu, après un éventuel préchauffage, les corps nécessaires à la réaction de conversion seront tout d'abord admis en partie supérieure de la chambre 6 de mélange/réaction dans laquelle ils débouchent à co-courants les uns des autres, dans le sens de la flèche 20, avec, en allant de l'intérieur du réacteur vers l'extérieur, le premier gaz "riche" en oxygène, puis, l'entourant de façon sensiblement contiguë, le second gaz "riche" en hydrogène, lui-même entouré, également de façon sensiblement contiguë, par la charge hydrocarbonée et éventuellement un flux de vapeur d'eau.

Vers l'entrée de la chambre 6 de mélange/réaction et tandis qu'ils commencent à se mélanger, ces différents corps sont alors, sensiblement à un même niveau donné de circulation, enflammés par les moyens d'allumage 7, de sorte à déclencher l'opération de conversion. Une flamme pilote (non représentée) hydrogène/oxygène devrait permettre de façon commode d'assurer le maintien et la régulation de cet allumage.

Bien qu'elles soient difficiles à préciser et qu'elles dépendent des conditions opératoires, les températures de conversion des hydrocarbures devraient être, en partie haute de la chambre 6, comprises entre environ 800 et 1600°C.

Quoi qu'il en soit, on maintiendra d'une façon générale la température des parois de la chambre en question inférieure à 1100°C environ, par exemple par injection de vapeur d'eau, en adaptant la quantité d'oxygène admise, ou encore en préchauffant l'hydrocarbure à convertir dans les chambres 15, 15′. Les temps de séjour des corps dans cette chambre 6 seront en général compris entre 2 ms et 0,5 s environ et, de préférence, entre 10 et 100 ms.

Parvenus dans la partie basse du réacteur, où la température atteindra en général 200 à 500°C environ, les produits de conversion subiront alors,dans la chambre 9 et selon une technique classique, une trempe au contact du fluide de trempe (tel que de l'huile) débouchant des buses 10, de sorte à atteindre sensiblement la température ambiante.

Dans le cas où le type d'hydrocarbure(s) que l'on désire convertir ne sera pas constitué essentiellement (c'est-à-dire à plus de 50 % environ) d'hydrocarbure(s) dit(s) "léger(s)", c'est-à-dire comprenant entre deux et cinq atomes de carbone, on notera qu'en complément de cette trempe, il pourra s'avérer utile de prévoir une pré-trempe du mélange réactionnel. On introduira alors par les canaux 12 une telle charge hydrocarbonée "légère", fluide ou liquide, qui débouchera donc vers la partie intermédiaire de la chambre 6 et à courant croisé avec le mélange en réaction.

On notera qu'en utilisant une telle charge hydrocarbonée de poids moléculaire relativement faible, on augmentera la quantité d'hydogène en sortie du réacteur.

Quoi qu'il en soit, une fois stabilisés par la trempe, les hydrocarbures de conversion seront canalisés en partie basse de la chambre 9 et récupérés par les conduits 11 d'évacuation (voir figure 1).

Revenons maintenant quelque peu sur les caractéristiques des corps réactifs utilisés, de façon à noter tout d'abord qu'éventuellement le premier gaz riche en oxygène et qui circule dans la veine centrale 1 du réacteur peut contenir une certaine quantité d'hydrogène, l'hydrocarbure pouvant, quant à lui, être constitué d'un mélange d'hydrocarbures ou encore contenir une certaine quantité de vapeur d'eau (dans ce cas au moins 10 % en poids environ), voire même d'oxygène (dans ce cas au moins de l'ordre de 5 % en volume).

On notera néanmoins dès à présent que la quantité d'hydrogène en circulation sera de toute façon en excès par rapport à la quantité d'oxygène. Plus précisément, on prévoit selon l'invention que la quantité volumique d'hydrogène apportée par les premier et second gaz sera au moins deux fois, et de préférence quatre fois, supérieure à la quantité d'oxygène apportée par le premier gaz. En outre, la quantité d'hydrogène apportée par le second gaz représentera au moins 10 % , et de préférence 100 % , de la quantité totale d'hydrogène apportée par les premier et second gaz.

De cette façon, l'hydrogène jouera un rôle effectif d'isolant "chimique" entre le premier gaz riche en oxygène et la charge hydrocarbonée. En outre, cet hydrogène en excès pourra intervenir en favorisant notamment la dilution des corps en réaction et le contrôle de l'élévation de température une fois le réacteur allumé.

Il a également été remarqué que cet excès voulu d'hydrogène permettait d'améliorer la sélectivité de la conversion de l'hydrocarbure initial à transformer et réduisait donc la formation de suies et autres dépôts indésirables, tout en favorisant la formation d'hydrocarbures convertis "moins insaturés" (tels que l'éthylène, le propène...) au détriment des hydrocarbures convertis "moins saturés" (tels que l'acétylène).

En ce qui concerne l'enthalpie libre nécessaire à la conversion de ces hydrocarbures, on notera qu'elle est globalement fournie par l'oxygène véhiculé dans le premier gaz et éventuellement dans la charge hydrocarbonée à transformer. Dans ce dernier cas, on remarquera que la possibilité offerte de régler indépendamment les alimentations d'oxygène apportées par les conduits 1 (gaz riche en oxygène) et 3 (hydrocarbure), procurera une souplesse accrue de mise en oeuvre du procédé, en fonction de la nature de la charge hydrocarbonée de départ et des produits de conversion que l'on désire obtenir. A ce sujet, il a été remarqué que le réglage de la quantité d'oxygène véhiculée par l'hydrocarbure influait sur les températures de réaction, permettant d'ajuster la quantité d'oxyde de carbone produite, ainsi que la quantité d'hydrogène sortant, après réaction, du réacteur et d'obtenir un fonctionnement réellement "autothermique" du procédé, c'est-à-dire tel que tous les corps entrent dans les chambres de préchauffage 15, 15′ sensiblement à température ambiante.

Comme on le voit, le procédé de l'invention se différencie nettement des procédés de conversion connus en un ou deux stades précités.

Si l'on cherche à préciser ce point, on peut en particulier relever que l'oxygène contenu dans le premier gaz est, conformément au mode de réalisation retenu et au moment de l'inflammation des corps dans la chambre 6 de réaction/mélange, séparé de la charge hydrocarbonée par le second gaz riche en hydrogène. Il ne s'agit donc pas ici d'un procédé en un stade caractérisé par le prémélange de l'oxygène et de l'hydrocarbure avant allumage. En outre, le corps gazeux résultant du mélange et de la réaction de combustion entre l'hydrogène et l'oxygène est, dans l'invention, mélangé en quelque sorte "en l'état" à la charge hydrocarbonée, sans avoir subi d'ajout préalable de vapeur d'eau, comme c'est le cas dans les procédés connus en deux stades.

De plus, les expériences menées ont montré que compte tenu de la géométrie du réacteur et du rapport surface mouillée sur volume de la chambre de mélange/réaction compris entre 40 et 250 m$^{-1}$, le mélange des corps dans leur ensemble s'effectuait largement par diffusion, caractéristique considérée jusqu'à présent comme rédhibitoire.

Ces mêmes expériences ont permis de mettre en évidence que la quantité d'hydrogène sortant du réacteur pouvait être sensiblement la même qu'à l'entrée, ce qui est tout à fait favorable dans le sens où l'hydrogène apparaîtrait alors comme un véritable initiateur ou "catalyseur" homogène des réactions de conversion des charges hydrocarbonées. En pratique, on tendra d'autant plus vers ce type de fonctionnement du réacteur qu'on augmentera la quantité d'oxygène apportée par le flux d'hydrocarbure et qu'on procédera à un préchauffage de tout ou partie des corps devant réagir. En pratique, le rapport oxygène/hydrocarbure entrant dans le réacteur sera essentiellement adapté en fonction du taux de conversion (c'est-à-dire de la quantité d'hydrocarbure converti sur la quantité d'hydrocarbure entrant) et de la sélectivité recherchés.

Quel que soit le dosage retenu de ces corps, un tel préchauffage pourra, comme on l'a vu, consister en une circulation, en particulier de la charge hydrocarbonée, à contre-courant des corps en réaction, le long des parois extérieures de la chambre 6 de mélange/réaction ; ce transfert de chaleur induisant en quelque sorte une première trempe (ou refroidissement) des corps en réaction dans la chambre.

Toutefois on pourra envisager, de façon complémentaire, un préchauffage annexe, notamment de la charge précitée, au niveau d'échangeurs classiques tels que 21 et dans lesquels circuleraient les corps à préchauffer avant d'arriver au réacteur.

Quoi qu'il en soit, on comprendra que le préchauffage par circulation le long de la face extérieure des parois de la chambre 6 sera largement favorisé par la possible utilisation de parois entièrement métalliques (tel que de l'acier 25 Cr/20 Ni ou 25 Cr/35 Ni/1,5 Nb), au contraire des appareils connus dont les parois sont habituellement en matériaux réfractaires.

Bien entendu, cette possible réalisation métallique du réacteur découle, pour une part importante, du fait que la réaction fortement exothermique entre l'oxygène et l'hydrogène se déroule dans la zone centrale du réacteur, tandis que celle endothermique de transformation des hydrocarbures se déroule dans une zone périphérique, proche des parois de la chambre 6, l'hydrocarbure jouant alors son rôle d'isolant thermique pour la paroi du réacteur. Concernant cette protection thermique, il est bien évident qu'elle sera accrue si l'on fait circuler, débouchant de la veine 4, de la vapeur d'eau (pouvant contenir une petite quantité d'huile en dispersion) le long de la face intérieure des parois de la chambre.

On notera que cette barrière thermique pourrait même être encore améliorée en prémélangeant de l'eau sous forme de vapeur à la charge hydrocarbonée circulant dans la veine 3.

En ce qui concerne l'utilité de cette vapeur d'eau en présence dans le mélange réactionnel, qu'il s'agisse de celle amenée de l'extérieur par les veines d'alimentation ou de celle produite in-situ par les réactions chimiques, il semble important de relever qu'elle intervient non seulement en influant sur les températures et les pressions partielles régnant dans la réacteur, mais également en réduisant les dépôts de suies et par son effet oxydant bénéfique pour les parois métalliques de ce même réacteur.

Bien que cela n'ait pas été précisé jusqu'à présent, ni représenté, il est bien entendu qu'en fin d'opération de trempe, après stabilisation des produits de transformation obtenus, on peut récupérer et recycler une partie au moins d'entre-eux vers les veines d'admission correspondantes.

A ce sujet, il est clair que les modalités de recyclage des produits sortant du réacteur (en particulier l'hydrogène, l'oxyde de carbone, la part d'hydrocarbure(s) non converti(s)) sont particulièrement souples. Plus précisément, ces modalités de recyclage peuvent aller d'un premier cas limite comportant comme connu en soi, la séparation de l'oxyde de carbone, de l'hydrogène et l'hydrocarbure de départ récupéré, la transformation de cet oxyde de carbone en hydrogène, le recyclage de l'hydrogène dans la veine 2 (et éventuellement 1) et de l'hydrocarbure considéré dans la veine 3, à un autre cas limite où le mélange oxyde de carbone/hydrogène/hydrocarbure de départ récupéré est recyclé tel quel, sans fractionnement, dans les courants des veines correspondantes. Dans ce même esprit, on pourrait également fractionner, selon un procédé en soi connu, une partie des hydrocarbures sortant du réacteur de façon à les recycler dans la veine 3 et, si nécessaire, dans les conduits 12.

De façon à éclairer aux mieux les conditions d'application de l'invention, on va maintenant présenter quelques exemples opératoires.

EXEMPLE 1

On alimente les veines 1, 2, 3 respectivement en oxygène, hydrogène et méthane.

Les proportions volumiques de ces trois gaz sont les suivantes : 0,75/3/1.

On effectue un préchauffage des gaz en les faisant circuler en 15, 15′ le long de la paroi extérieure de la chambre de mélange/réaction du réacteur.

A leur introduction dans la chambre, l'inflammation est amorcée et la réaction de conversion déclenchée. La température de conversion du méthane dans la chambre de mélange/réaction est de l'ordre de 1300 à 1400°C.

Après trempe du mélange réactif résultant, on obtient les corps carbonés suivants : acétylène, éthylène, éthane, oxyde de carbone, gaz carbonique, ainsi que du méthane et de l'hydrogène.

Le temps de séjour moyen des corps dans le réacteur a été d'environ quarante millisecondes.

EXEMPLE 2

On répète le mode opératoire de l'exemple 1 en ajoutant de l'oxygène au méthane, selon une proportion volumique de 10 % environ.

On observe alors, en comparaison de l'exemple 1, une formation accrue d'hydrocarbures insaturés (acétylène), d'oxyde de carbone et d'hydrogène.

EXEMPLE 3

On répète le mode opératoire de l'exemple 1 et l'on introduit parallèlement par les canaux 12 de l'éthane, en quantité sensiblement égale à celle du méthane.

Comparé aux résultats de l'exemple 1, on observe un accroissement des quantités d'éthylène et d'acétylène obtenues, de même pour l'hydrogène.

EXEMPLE 4

On répète le mode opératoire de l'exemple 3 en remplaçant l'éthane par du propane.

Outre les produits résultants obtenus dans l'exemple 3, on observe la formation de propène.

EXEMPLE 5

Après un préchauffage de la charge hydrocarbonée par circulation dans l'échangeur-préchauffeur 21 et par échange thermique avec les parois du réacteur (dans les chambres 15, 15′), on introduit dans la chambre 6 du réacteur
– débouchant de la veine 1 : de l'oxygène,
– débouchant de la veine 2 : du gaz de four à coke contenant environ 60 % d'hydrogène, 30 % de méthane, ainsi que d'autres hydrocarbures, de l'oxyde de carbone, du gaz carbonique et de l'azote,
– débouchant de la veine 3 : du gaz naturel contenant environ 80 % de méthane, ainsi que de l'éthane, du propane et du butane.

En proportion, les quantités volumiques entre les corps qui circulent dans les trois veines sont : 0,35/1/0,5.

Après trempe, les produits de réaction obtenus contiennent de l'acétylène, de l'éthylène, de l'oxyde de carbone et du gaz carbonique, ainsi que de petites quantités de diverses mono-oléfines, di-oléfines, acétyléniques et des hydrocarbures saturés.

EXEMPLE 6

On répète le mode opératoire de l'exemple 5 en remplaçant le gaz de four à coke par un gaz dit de synthèse contenant environ 75 % d'hydrogène et 25 % d'oxyde de carbone.

En proportion, les quantités volumiques entre les corps qui circulent dans les trois veines sont ici de 0,3/1/0,45.

Après trempe, on obtient un mélange de gaz de même nature que dans l'exemple 5.

EXEMPLE 7

On introduit après préchauffage par échange thermique avec les parois du réacteur
– par la veine 1, de l'oxygène
– par la veine 2, de l'hydrogène
– et par la veine 3, un mélange de vapeur d'eau et d'hydrocarbures dans un rapport massique égal à 0,65. La charge hydrocarbonée est ici constituée d'un mélange de gazole légers et de gazole lourds. Sa formule brute est $CH_{1,86}S_{0,004}$. En proportion, les quantités volumiques des différents corps en circulation dans les trois veines sont 4/1/2,4.
– après trempe du mélange réactif, on obtient un mélange complexe d'hydrocarbures saturés (incluant le méthane) et insaturés (mono-oléfines, di-oléfines, acétylèniques), d'hydrocarbures benzèniques, d'oxyde de carbone, de gaz carbonique, d'anhydride sulfureux et d'hydrogène.

Bien que la description qui précède n'ait été faite en référence essentiellement qu'à un mode particulier de réalisation, il doit être clair que l'invention ne se limite pas exclusivement à celui-ci. Différentes variantes de réalisation pourraient être envisagées.

En particulier, à proximité de l'endroit où elles s'ouvrent sur la chambre 6 de mélange/réaction, les diverses veines d'alimentation du réacteur pourraient être disposées de sorte qu'une veine donnée "n'entoure" une veine plus intérieure que sur deux côtés opposés, au lieu de l'entourer complètement sur toute sa périphérie, tel qu'illustré par exemple à la figure 2.

Par ailleurs, en ce qui concerne la géométrie du réacteur, on pourrait prévoir une chambre 6 de mélange/réaction, sensiblement cylindrique annulaire alimentée par des veines disposées coaxialement selon une géométrie également cylindrique annulaire.

Egalement, on aurait pu prévoir, au lieu d'un préchauffage annexe par des échangeurs 21 situés en amont des chambres 15, 15' (figure 1), de disposer ces mêmes échangeurs 21 juste en aval, à la sortie des chambres en question.

Enfin, bien que l'on ait envisagé plus particulièrement de fabriquer les parois de la chambre de mélange/réaction en métal, cela n'exclut en aucun cas l'utilisation d'autres matériaux et notamment les matériaux "nouveaux" tels que les céramiques.

**Revendications**

1. Procédé pour convertir des hydrocarbures en produits de conversion, caractérisé en ce que :
   a) - on utilise en tant que substances de départ au moins un type d'hydrocarbure et un premier gaz contenant au moins environ 20 % d'oxygène en volume,
   b) - on fait circuler ces substances indépendamment l'une de l'autre et suivant des flux à co-courants, sans mélange, en adoptant une répartition spatiale telle que ledit flux d'hydrocarbure soit situé autour du flux dudit premier gaz,
   c) - on laisse ces mêmes substances se mélanger à un premier niveau donné de circulation et on enflamme le mélange d'oxygène et d'hydrocarbure de manière à induire la réaction de conversion,
   d) - on conduit une trempe du mélange résultant converti, à partir d'un second niveau de circulation situé en aval du premier,
   e) - et on récupère les produits de conversion résultants trempés.

2. Procédé selon la revendication 1 caractérisé en ce que :
   – on fait en outre circuler un second gaz en tant que substance additionnelle de départ, ce second gaz contenant au moins environ 30 % en volume d'hydrogène et circulant entre les flux hydrocarbure et dudit premier gaz,
   – et on enflamme cet hydrogène à l'endroit dudit premier niveau de circulation où les substances se mélangent entre-elles.

3. Procédé selon la revendication 1 ou la revendication 2 caractérisé en ce que le flux d'hydrocarbure contient environ 10 % de vapeur d'eau en poids.

4. Procédé selon l'une quelconque des revendications précédentes caractérisé en ce que le flux d'hydrocarbure contient au moins environ 5 % en volume d'oxygène.

5. Procédé selon l'une quelconque des revendications précédentes caractérisé en ce que le premier gaz contient une quantité déterminée d'hydrogène.

6. Procédé selon la revendication 5 caractérisé en ce que la quantité d'hydrogène en volume contenue dans le second gaz est égale à au moins 10 % de la quantité d'hydrogène totale en volume contenue dans lesdits premier et second gaz.

7. Procédé selon l'une quelconque des revendications 2 à 6 caractérisé en ce que la quantité totale d'hydrogène en volume est au moins deux fois, et de préférence quatre fois, supérieure à la quantité en volume d'oxygène.

8. Procédé selon l'une quelconque des revendications précédentes caractérisé en ce que, pour tremper le mélange résultant converti à partir dudit second niveau de circulation, on conduit un échange thermique indirect, sans qu'il y ait mélange, entre ledit mélange résultant converti et une partie au moins desdites substances de départ que l'on fait circuler, à contre-courant, autour dudit mélange résultant converti de manière, à assurer concomitamment ladite trempe et le préchauffage desdites substances avant qu'elles ne circulent en flux à co-courants suivant l'étape b) de la revendication 1.

9. Procédé selon la revendication 8 caractérisé en ce que, pour compléter ladite trempe par échange thermique indirect, on injecte un fluide de trempe dans ledit mélange résultant converti.

10. Procédé selon l'une quelconque des revendications précédentes caractérisé en ce que, dans le cas où ledit hydrocarbure de départ comprend moins de deux ou plus de cinq atomes de carbone, pour tremper le mélange résultant converti, on introduit dans ce dernier, suivant une direction transversale par rapport au courant général de circulation du flux desdites substances de départ, au moins un autre hydrocarbure comprenant entre deux et cinq atomes de carbone.

11. Procédé selon l'une quelconque des revendications précédentes caractérisé en ce qu'on admet de la vapeur d'eau dans la chambre de mélange et de réaction suivant un flux indépendant sensiblement parallèle au flux de l'hydrocarbure de départ, cette vapeur d'eau étant mélangée avec lesdites substances de départ au premier niveau précité de circulation.

12. Procédé selon la revendication 11 caractérisé en ce que la vapeur d'eau admise contient une dispersion d'huile.

13. Procédé selon l'une quelconque des revendications précédentes caractérisé en ce qu'on recycle une partie au moins des produits de conversion résultants trempés récupérés lors de l'étape e) de la revendication 1 pour les incorporer aux substances de départ.

14. Appareil pour conduire une réaction de conversion exothermique entre au moins un premier et un second réactifs, ledit appareil comprenant :
– une chambre de mélange et de réaction (6) pour y mélanger et y enflammer au moins lesdits premier et second réactifs de manière à conduire la réaction exothermique de conversion, ladite chambre étant limitée extérieurement par une paroi réalisée au moins partiellement en matériau thermiquement conducteur, pour transférer la chaleur de la réaction de conversion à travers cette paroi,
– des moyens d'allumage (7) pour induire la réaction de conversion au moins entre lesdits premier et second réactifs,
– un premier conduit d'alimentation (1) communiquant avec la chambre de mélange et de réaction pour y introduire, à une première extrémité, ledit premier réactif,
– un second conduit d'alimentation (2) communiquant avec ladite chambre de mélange et de réaction pour introduire le second réactif à cette même première extrémité, ce second conduit d'alimentation s'étendant extérieurement le long dudit premier conduit, sensiblement parallèlement à ce dernier, au moins à proximité de ladite chambre de mélange et de réaction (6),
– des conduits de récupération (11) communiquant avec la chambre de mélange et de réaction pour récupérer les produits de réaction résultants,
– des chambres de préchauffage (15) communiquant avec l'un au moins desdits premier et second conduits d'alimentation et étant alimentées avec l'un au moins desdits réactifs, ces chambres de préchauffage s'étendant extérieurement au contact de ladite paroi extérieure thermiquement conductrice de la chambre de mélange et de réaction pour préchauffer le, ou les, réactif(s) circulant dans ces chambres et pour fournir à la chambre de mélange et de réaction une première zone de trempe interne, par transfert de chaleur de la réaction de conversion vers le, ou les, réactif(s) circulant dans lesdites chambres (15, 15') de préchauffage, avant que lesdits réactifs soient admis dans les conduits d'alimentation (1, 2, 3 4).

15. Appareil selon la revendication 14 caractérisé en ce qu'il comprend en outre des conduits (10) pour alimenter avec un fluide de trempe la chambre de mélange et de réaction, de manière à y créer une seconde zone de trempe (9) située entre ladite première zone et lesdits conduits (11) de récupération des produits de réaction, assurant ainsi une trempe supplémentaire de ces mêmes produits de réaction avant leur récupération.

16. Appareil selon la revendication 14 ou la revendication 15 caractérisé en ce que le rapport de la surface mouillée sur le volume de ladite chambre de mélange et de réaction est compris entre environ 40 et environ 250 m$^{-1}$.

17. Appareil selon l'une quelconque des revendications 14 à 16 caractérisé en ce que la chambre de mélange et de réaction présente une section sensiblement rectangulaire.

18. Appareil selon l'une quelconque des revendications 14 à 17 caractérisé en ce qu'il comprend en outre au moins un troisième conduit d'alimentation (3) communiquant avec ladite chambre de mélange et de réaction pour introduire à ladite première extrémité de celle-ci un troisième réactif, ce troisième conduit d'alimentation s'étendant extérieurement le long dudit second conduit (2), sensiblement parallèlement à ce même conduit au moins à proximité de ladite chambre de mélange et de réaction (6).

19. Appareil selon l'une quelconque des revendications 15 à 18 caractérisé en ce que des canaux (12) d'alimentation en hydrocarbure(s) supplémentaire(s) débouchent sensiblement perpendiculairement à une paroi latérale extérieure de la chambre de mélange et de réaction, en un endroit de cette dernière qui est intermédiaire entre la zone où lesdits conduits d'alimentation (1, 2, 3, 4) communiquent avec cette même chambre et ladite seconde zone de trempe (9) de cette dernière.

**Patentansprüche**

1. Verfahren zur Umsetzung von Kohlenwasserstoffen in Umsetzungsprodukte, dadurch gekennzeichnet, daß:

a) - man als Ausgangssubstanzen mindestens einen Kohlenwasserstofftyp und ein erstes Gas verwendet, welches mindestens etwa 20 Vol.-% Sauerstoff enthält,

b) - man diese Substanzen unabhängig voneinander zirkulieren läßt und den gleichgerichteten Strömen ohne Mischen folgend eine räumliche Aufteilung derart ausführt, daß z.B. der Kohlenwasserstoffstrom um den Fluß des ersten Gases herum angeordnet ist,

c) - man eben diese Substanzen sich an einer ersten gegebenen Zirkulationsstelle mischen läßt und man das Gemisch von Sauerstoff und Kohlenwasserstoff derart entflammt, daß man die Umsetzungsreaktion einleitet,

d) - man ein Abschrecken des sich ergebenden, umgesetzten Gemisches durchführt, ausgehend von einer zweiten Zirkulationsstelle, die abstromig von der ersten angeordnet ist, und

e) - man die sich ergebenden, abgeschreckten Umsetzungsprodukte wiedergewinnt.

2. Verfahren nach Anspruch 1, dadurch gekennzeichnet, daß:

– man ferner ein zweites Gas als zusätzliche Ausgangssubstanz zirkulieren läßt, wobei dieses zweite Gas mindestens etwa 30 Vol.-% Wasserstoff enthält und zwischen den Kohlenwasserstoffströmen und dem ersten Gas zirkuliert,

– und man diesen Wasserstoff an der Stelle der ersten Zirkulationsstelle entflammt, wo sich die Substanzen miteinander mischen.

3. Verfahren nach Anspruch 1 oder Anspruch 2, dadurch gekennzeichnet, daß der Kohlenwasserstoffstrom etwa 10 Gew.-% Wasserdampf enthält.

4. Verfahren nach einem der vorhergehenden Ansprüche, dadurch gekennzeichnet, daß der Kohlenwasserstoffstrom mindestens etwa 5 Vol.-% Sauerstoff enthält.

5. Verfahren nach einem der vorhergehenden Ansprüche, dadurch gekennzeichnet, daß das erste Gas eine bestimmte Menge Wasserstoff enthält.

6. Verfahren nach Anspruch 5, dadurch gekennzeichnet, daß die Volumenmenge an Wasserstoff, die in dem zweiten Gas enthalten ist, gleich mindestens 10 % der gesamten Volumenmenge an Wasserstoff ist, die in dem ersten und zweiten Gas enthalten ist.

7. Verfahren nach einem der Ansprüche 2 bis 6, dadurch gekennzeichnet, daß die gesamte Volumenmenge an Wasserstoff mindestens um das Zweifache, vorzugsweise um das Vierfache höher ist als die Volumenmenge an Sauerstoff.

8. Verfahren nach einem der vorhergehenden Ansprüche, dadurch gekennzeichnet, daß zum Abschrecken des sich ergebenden, umgesetzten Gemisches, ausgehend von der zweiten Zirkulationsstelle, man einen indirekten Wärmeaustausch durchführt, ohne daß ein Mischen stattfindet zwischen dem sich ergebenden, umgesetzten Gemisch und einem Teil mindestens der Ausgangssubstanzen, die man im Gegenstrom um das sich ergebende, umgesetzte Gemisch derart zirkulieren läßt, daß gleichzeitig das Abschrecken und das Vorerwärmen der Substanzen sichergestellt ist, bevor sie im Gleichstrom nach Stufe b) des Anspruches 1 zirkulieren.

9. Verfahren nach Anspruch 8, dadurch gekennzeichnet, daß zur Vervollständigung des Abschreckens durch indirekten Wärmeaustausch man ein Abschreckfließmittel in das sich ergebende, umgesetzte Gemisch einspritzt.

10. Verfahren nach einem der vorhergehenden Ansprüche, dadurch gekennzeichnet, daß in dem Falle, wo der Ausgangskohlenwasserstoff weniger als zwei oder mehr als fünf Kohlenstoffatome aufweist, man zum Abschrecken des sich ergebenden, umgesetzten Gemisches in dieses letztere in Richtung quer zum allgemeinen Fließen des Zirkulationsstroms der Ausgangssubstanzen mindestens einen anderen Kohlenwasserstoff einführt, der zwischen zwei und fünf Kohlenstoffatome enthält.

11. Verfahren nach einem der vorhergehenden Ansprüche, dadurch gekennzeichnet, daß man Wasserdampf in die Misch- und Reaktionskammer gemäß einem unabhängigen Strom einführt, der im wesentlichen parallel zum Strom des Ausgangskohlenwasserstoffes ist, wobei dieser Wasserdampf mit den Ausgangssubstanzen an der genannten ersten Zirkulationsstelle gemischt wird.

12. Verfahren nach Anspruch 11, dadurch gekennzeichnet, daß der zugeführte Wasserdampf eine Öldispersion enthält.

13. Verfahren nach einem der vorhergehenden Ansprüche, dadurch gekennzeichnet, daß man mindestens einen Teil der sich ergebenden, abgeschreckten, während der Stufe e) des Anspruches 1 wiedergewonnenen Umsetzungsprodukte rezykliert, um sie in die Ausgangssubstanzen einzubauen.

14. Vorrichtung zur Durchführung einer exothermen Umsetzungsreaktion zwischen mindestens einem ersten und einem zweiten Reagenz, wobei die Vorrichtung aufweist:

– eine Misch- und Reaktionskammer (6), um dort mindestens das erste und zweite Reagenz derart zu mischen und zu entflammen, daß die exotherme Umsetzungsreaktion durchgeführt wird, wobei diese Kammer nach außen durch eine Wand begrenzt ist, die mindestens teilweise aus einem wärmeleitenden Material hergestellt ist, um die Wärme der Umsetzungsreaktion durch diese Wand hindurchzuleiten,

– Zündmittel (7), um die Umsetzungsreaktion mindestens zwischen diesem ersten und zweiten Reagenz einzuleiten,

– eine erste Versorgungsleitung (1), die mit der Misch- und Reaktionskammer verbunden ist, um dort an einem ersten Ende das erste Reagenz einzuführen,

– eine zweite Versorgungsleitung (2), die mit der Misch- und Reaktionskammer verbunden ist, um das zweite Reagenz an eben diesem ersten Ende einzuführen, wobei diese zweite Versorgungsleitung sich nach außen längs der ersten Leitung erstreckt, im wesentlichen parallel zu letzterer und mindestens in die Nachbarschaft der Misch- und Reaktionskammer (6),

– Wiedergewinnungsleitungen (11), die mit der Misch- und Reaktionskammer in Verbindung stehen, um die sich ergebenden Reaktionsprodukte wiederzugewinnen,

– Vorwärmkammern (15), die mit mindestens einer der ersten und zweiten Versorgungsleitungen in Verbindung stehen und mit mindestens einem der Reagenzien versorgt werden, wobei diese Vorwärmkammern sich nach außen in Berührung mit der außen wärmeleitfähigen Wand der Misch- und Reaktionskammer erstrecken, um das oder die in diesen Kammern zirkulierende(n) Reagenz(ien) vorzuwärmen und um die Misch- und Reaktionskammer mit einer ersten inneren Abschreckungszone zu versehen durch Wärmeübergang von der Umsetzungsreaktion zu dem oder den Reagenzien, die in diesen Vorwärmkammern (15, 15') zirkulieren, bevor die Reagenzien in die Versorgungsleitungen (1, 2, 3, 4) geführt werden.

15. Vorrichtung nach Anspruch 14, dadurch gekennzeichnet, daß sie ferner Leitungen (10) aufweist, um die Misch- und Reaktionskammer derart mit einem Abschreckfließmittel zu versorgen, daß dort eine zweite Abschreckzone (9) gebildet wird, die zwischen der ersten Zone und den Wiedergewinnungsleitungen (11) für die Reaktionsprodukte angeordnet ist, wobei also eine zusätzliche Abschreckung eben dieser Reaktionsprodukte vor ihrer Wiedergewinnung sichergestellt wird.

16. Vorrichtung nach Anspruch 14 oder Anspruch 15, dadurch gekennzeichnet, daß das Verhältnis benetzte Oberfläche zum Volumen der Misch- und Reaktionskammer zwischen etwa 40 und etwa 250 $m^{-1}$ liegt.

17. Vorrichtung nach einem der Ansprüche 14 bis 16, dadurch gekennzeichnet, daß die Misch- und Reaktionskammer einen im wesentlichen rechteckigen Querschnitt aufweist.

18. Vorrichtung nach einem der Ansprüche 14 bis 17, dadurch gekennzeichnet, daß sie ferner mindestens eine dritte Versorgungsleitung (3) aufweist, die mit der Misch- und Reaktionskammer verbunden ist, um in das erste Ende derselben ein drittes Reagenz einzuführen, wobei diese dritte Versorgungsleitung sich außen längs der zweiten Leitung (2) und im wesentlichen parallel zu dieser Leitung mindestens in die Nachbarschaft der Misch- und Reaktionskammer (6) erstreckt.

19. Vorrichtung nach einem der Ansprüche 15 bis 18, dadurch gekennzeichnet, daß Versorgungskanäle (12) für zusätzlichen Kohlenwasserstoff im wesentlichen senkrecht zu einer Außenseitenwand der Misch- und Reaktionskammer an einer Stelle der letzteren münden, die sich zwischen der Zone, wo die Versorgungsleitungen (1, 2, 3, 4) mit eben dieser Kammer in Verbindung stehen, und der zweiten Abschreckungszone (9) der letzteren befindet.

## Claims

1. Process for converting hydrocarbons into conversion products, characterised in that:

a) at least one type of hydrocarbon and a first gas containing at least approximately 20 volume percent of oxygen are used as initial substances;

b) these substances are circulated independently of one another and in co-current flows without being mixed and are distributed spatially such that the said hydrocarbon flow is located around the flow of the said first gas;

c) these same substances are allowed to mix at a first given circulation level and the mixture of oxygen and hydrocarbon is ignited so as to induce the conversion reaction;

d) the resultant converted mixture is tempered from a second circulation level located downstream of the first;

e) and the resultant tempered conversion products are recovered.

2. Process according to Claim 1, characterised in that a second gas is also circulated as an additional initial substance, this second gas containing at least approximately 30 volume percent hydrogen and circulating between the hydrocarbon flow and the flow of the said first gas; and this hydrogen is ignited at the location of the said first circulation level where the substances are mixed with one another.

3. Process according to Claim 1 or Claim 2, characterised in that the hydrocarbon flow comprises approximately 10 weight percent water vapour.

4. Process according to any one of the preceding claims, characterised in that the hydrocarbon flow contains at least approximately 5 volume percent oxygen.

5. Process according to any one of the preceding claims, characterised in that the first gas contains a given amount of hydrogen.

6. Process according to Claim 5, characterised in that the amount of hydrogen by volume in the second gas is at least 10 volume percent of the total amount of hydrogen by volume in the said first and second gases.

7. Process according to any one of Claims 2 to 6, characterised in that the total amount of hydrogen by volume is at least two times, and preferably four times, greater than the amount of oxygen by volume.

8. Process according to any one of the preceding claims, characterised in that, in order to temper the resultant converted mixture from the second circulation level, indirect heat exchange is performed, without any mixing, between the said resultant converted mixture and at least some of the said initial substances which are circulated in counter-current about the said resultant converted mixture in order that the said substances are concomitantly tempered and preheated before they circulate in co-current flows according to stage b) of Claim 1.

9. Process according to Claim 8, characterised in that a tempering fluid is injected into the said resultant converted mixture in order to complete the said tempering by indirect heat exchange.

10. Process according to any one of the preceding claims, characterised in that if the said initial hydrocarbon comprises less than two or more than five carbon atoms, in order to temper the resultant converted mixture, at least one further hydrocarbon comprising between two and five carbon atoms is injected into the said resultant converted mixture in a transverse direction relative to the general circulation current of the flow of the said initial substances.

11. Process according to any one of the preceding claims, characterised in that water vapour is introduced into the mixing and reaction chamber in an independent flow substantially parallel to the initial hydrocarbon flow, this water vapour being mixed with the said initial substances at the aforementioned first circulation level.

12. process according to Claim 11, characterised in that the water vapour introduced contains a dispersion of oil.

13. Process according to any one of the preceding claims, characterised in that at least some of the tempered resultant conversion products recovered during stage e) of Claim 1 are recycled in order to be incorporated in the initial substances.

14. Apparatus for performing an exothermic conversion reaction between at least one first and one second reagent, the said apparatus comprising:
  – a mixing and reaction chamber (6) for mixing and igniting there at least the said first and second reagents so as to perform the exothermic conversion reaction, the said chamber being limited externally by a wall formed at least partially of heat-conductive material, in order to transfer the heat from the conversion reaction through this wall;
  – ignition means (7) for inducing the conversion reaction at least between the said first and second reagents;
  – a first feed duct (1) communicating with the mixing and reaction chamber in order to introduce thereinto, at a first end, the said first reagent;
  – a second feed duct (2) communicating with the said mixing and reaction chamber in order to introduce the second reagent at the same first end, this second feed duct extending externally along the said first duct, substantially parallel thereto, at least in the vicinity of the said mixing and reaction chamber (6);
  – recovery ducts (11) communicating with the mixing and reaction chamber in order to recover the resultant reaction products;
  – pre-heating chambers (15) communicating with at least one of the said first and second feed ducts and being supplied with at least one of the said reagents, these pre-heating chambers extending externally in contact with the said heat-conductive external wall of the mixing and reaction chamber in order these pre-heat the reagent or reagents circulating in these chambers and in order to provide the mixing and reaction chamber with a first internal tempering zone, by transferring the head from the conversion reaction towards the reagent or reagents circulating in the said pre-heating chambers (15, 15′), before the said reagents are introduced into the feed ducts (1, 2, 3, 4).

15. Apparatus according to Claim 14, characterised in that it further comprises ducts (10) for supplying the mixing and reaction chamber with tempering fluid so as to create therein a second tempering zone (9) located between the first zone and the said ducts (11) for recovering the reaction products, thus providing an additional tempering process for these same reaction products before they are recovered.

16. Apparatus according to Claim 14 or 15, characterised in that the ratio between the wet surface and the volume of the said mixing and reaction chamber is between approximately 40 and approximately 250 m$^{-1}$.

17. Apparatus according to any one of Claims 14 to 16, characterised in that the mixing and reaction chamber has a substantially rectangular cross-section.

18. Apparatus according to any one of Claims 14 to 17, characterised in that it further comprises at least a third feed duct (3) communicating with the said mixing and reaction chamber to introduce at the first end thereof a third reagent, this third feed duct extending externally along the second duct (2), substantially parallel to this same duct at least in the vicinity of the said mixing and reaction chamber (6).

19. Apparatus according to any one of Claims 15 to 18, characterised in that ducts (12) for supplying additional hydrocarbon(s) open substantially perpendicularly into an external lateral wall of a mixing and reaction chamber at a point thereof which is between the zone where the said feed ducts (1, 2, 3, 4) communicate with this same chamber and the said second tempering zone (9) of the latter.

FIG. 1

FIG.2

FIG.3

FIG_4

FIG.5